(19) Europäisches Patentamt
European Patent Office
Office européen des brevets

(11) Publication number: **0 468 821 A1**

(12) **EUROPEAN PATENT APPLICATION**

(21) Application number: **91306886.2**

(22) Date of filing: **26.07.91**

(51) Int. Cl.[5]: **C07D 311/82,** C07D 493/10

(30) Priority: **27.07.90 JP 200905/90**

(43) Date of publication of application:
**29.01.92 Bulletin 92/05**

(84) Designated Contracting States:
**DE GB**

(71) Applicant: **TAOKA CHEMICAL CO., LTD**
**No. 2-11, Nishimikuni 4-chome**
**Yodogawa-ku Osaka-shi Osaka-fu (JP)**

(72) Inventor: **Aburada, Koji c/o Taoka Chem Co. Ltd**
**2-11, Nishimikuni 4-chome Yodogawa-ku**
**Osaka-shi Osaka-fu (JP)**
Inventor: **Akagi, Minoru c/o Taoka Chem Co. Ltd**
**2-11, Nishimikuni 4-chome Yodogawa-ku**
**Osaka-shi Osaka-fu (JP)**

(74) Representative: **Crisp, David Norman et al**
**D. YOUNG & CO. 10 Staple Inn**
**London, WC1V 7RD (GB)**

(54) **Process for the preparation of rhodamines.**

(57) A process for preparing rhodamines represented by the following general formula (I) or (II):

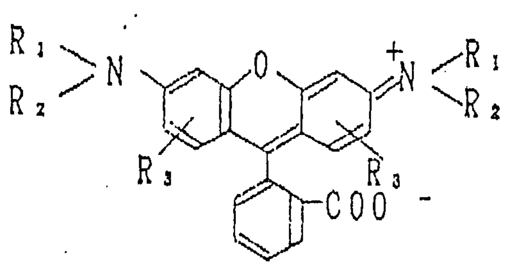

(I)

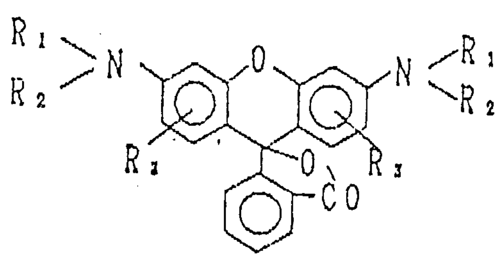

(II)

wherein $R_1$, $R_2$, and $R_3$ are independently hydrogen or a lower alkyl, group, but $R_1$ and $R_2$ are not both hydrogen which comprises subjecting phthalic anhydride and N-substituted-m-aminophenols represented by the following general formula (III):

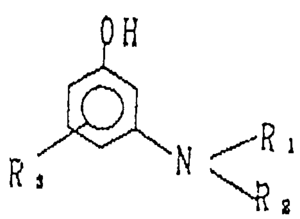

(III)

wherein $R_1$, $R_2$, and $R_3$ have the above-described meanings, to condensation reaction in one or more

EP 0 468 821 A1

organic solvents selected from the group consisting of halogentated and/or alkylated aromatic hydrocarbons or aliphatic hydrocarbons.

The present invention relates to an improvement in the industrial process for preparation of rhodamines.

Rhodamines such as rhodamine B, rhodamine 3B, rhodamine 6G, etc. are widely used as basic dyes, and a known process for preparation thereof is the melt condensation of N-alkyl-m-aminophenols with an excess of phthalic anhydride as described in, for example, "Yutaka HOSODA, Riron Seizo-Senryo Kagaku (Theory & Manufacture-Dye Chemistry), K.K. Gihodo, October 1963, pp. 373, 788" and others. This condensation reaction is carried out by slowly adding N-alkyl-m-aminophenols to molten phthalic anhydride. The condensation reaction mixture is discharged in alkaline water, such as an aqueous solution of sodium hydroxide, while hot and in a molten state, then after the complete removal of the excess phthalic anhydride the resulting solution is filtered once. The cake obtained is dissolved in an aqueous solution of hydrochloric acid, or the like, and then, crystallized out using e.g. hydrochloric acid, sodium chloride, etc. Thus, by filtration, washing, and drying of these crystals this process can produce the object rhodamine compounds.

In the conventional processes for preparation of rhodamines as above the phthalic anhydride is required in an amount corresponding to about 200 - 250 moles% of the theoretical amount for condensation, and the excess phthalic anhydride used can be removed by an aqueous alkali solution. However, the separation of the phthalic anhydride into the alkali solution is very inexpedient and two to three days are required for its complete removal. It has therefore been proposed that the amount of phthalic anhydride should be reduced as much as possible. However, reduction of the amount of the phthalic anhydride renders it difficult to maintain the phthalic acid in a molten state throughout the condensation process, so that this leads to poor operability as well as to a decrease in the yield and an increase in the by-product formation. Moreover, the filtration step after the removal of phthalic anhydride is also not free from defects in that handling of the cake gives rise to environmental problems such as dye pollution, and the like.

According to the present invention there is provided a process for preparing rhodamines represented by the general formula (I) or (II):

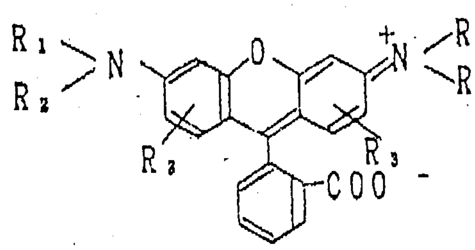

(I)

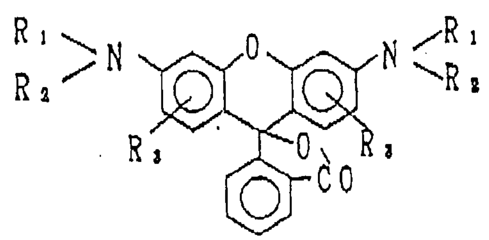

(II)

(wherein $R_1$, $R_2$, and $R_3$ are independently hydrogen or a lower alkyl group, but $R_1$ and $R_2$ are not both hydrogen) and salts and esters thereof which comprises subjecting phthalic anhydride and N-substituted-m-aminophenols represented by the following general formula (III):

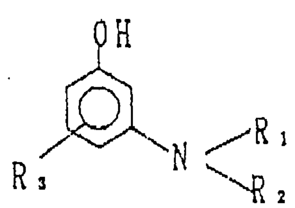

(III)

wherein $R_1$, $R_2$, and $R_3$ have the above-described meanings, to condensation reaction in one or more organic solvents selected from the group consisting of halogenated and/or alkylated aromatic hydrocarbons or aliphatic hydrocarbons. Preferably, in the present invention, lower alkyl is $C_{1-6}$ alkyl.

Various preferred features and embodiments of the present invention will now be described by way of non-limiting example.

According to the process of the present invention, the condensation reaction of phthalic anhydride with N-substituted-m-aminophenols represented by the above-described general formula (III) is carried out in one or more organic solvents selected from halogenated and/or alkylated aromatic hydrocarbons or aliphatic hydrocarbons.

There is no particular limitation on the organic solvent used in the present invention, but it is usually water-insoluble, and preferably is an organic solvent having a boiling point of about 170°C or higher. Preferred examples include o-dichlorobenzene, m-dichlorobenzene, p-dichlorobenzene, dichlorotoluene, trichlorobenzene, trichlorotoluene, iodobenzene, o-dibromobenzene, p-cymene, isobutylbenzene, sec-butylbenzene, nonylbenzene, octylbenzene, 1-decene, decane, etc. These organic solvents may be used singly or in mixtures of two or more thereof.

Preferred examples of N-substituted-m-aminophenol represented by the general formula (III) include mentioned N,N-dimethyl-m-aminophenol, N,N-dimethyl-m-aminophenol, N,N-dibutyl-m-aminophenol, o-(ethylamino)-p-cresol, etc.

The temperature of the condensation reaction by the process of the invention is usually about 160 - 220°C, and preferably about 175 -190°C. Further, in this process since use is made of a solvent or solvents, the fluidity of the condensation mixture is not a concern as in the prior art, and therefore, it is an advantage of the present invention that the raw materials can be charged all together at room temperature. However, as later described, for instance, in the case where dialkylaminophenols are used as the starting material, by-product formation often increases, so that it is not only preferred but also advantageous to reduce the amount of phthalic anhydride to be added that the starting materials are added in portions.

Furthermore, in the prior art, sulfuric acid is usually used for the purpose of improving the yield, or the like, at the time of condensation reaction, but in accordance with the process of the present invention high yields are obtained without the use of sulfuric acid, etc., and contrary to the teaching of the prior art, it was also found that sulfuric acid is quite useless since the use of sulfuric acid, etc. actually promotes the formation of impurities, and so on.

In addition, it was also found that in the condensation process of the present invention, an improvement in the yield can be achieved by carrying out the condensation reaction while passing an inert gas such as nitrogen, helium, etc. into the reaction system. This is also preferable from a safety aspect.

In addition, the present inventors further found that the yield and quality of the product can be effectively improved when the water formed in the condensation reaction is removed from the reaction system.

When the reaction is carried out while passing the above-described inert gas through the reaction system, the water formed is expelled from the system with said gas, and it is a great convenience.

A preferred extraction process for the rhodamines prepared according to the process of the present invention will now be described:

According to the present invention the reaction mixture obtained in the condensation reaction of the present invention may be subjected to extractive treatment with an aqueous solution of an alkali compound, and the excess of phthalic anhydride, etc. extracted in the aqueous layer is separated and can be removed.

Preferred alkali compounds include sodium hydroxide, potassium hydroxide, sodium carbonate, sodium bicarbonate, etc.

This operation, however, is required only for removal of phathalic anhydride when it has been used in excess, and hence it is not necessary when the theoretical amount of phthalic anhydride has been used.

In accordance with the above-described process the desired rhodamines are obtained as an organic solvent layer, and, by treating said organic solvent layer by mixing the dye material with an aqueous acid solution, the rhodamines, represented by the general formula (IV):

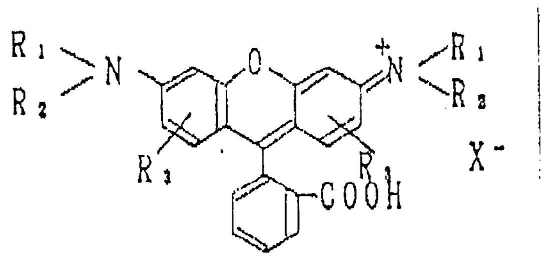

(IV)

wherein $R_1$, $R_2$, and $R_3$ have the above-described meanings, and X represents an anionic residue, can be readily obtained.

In the above case, preferred aqueous acid solutions include mineral acids such as sulfuric acid, hydrochloric acid, etc. and other inorganic acids, but mineral acids are particularly preferred.

The aqueous acidic solution of the rhodamines obtained in accordance with the above-described process may be subjected to crystallization by the usual methods using mineral acids or inorganic salts and after filtration, washing, and drying to obtain the desired rhodamines.

The rhodamines obtained in accordance with the above-described process may be esterified using conventional alkylating agents to readily produce rhodamines represented by the general formula (V):

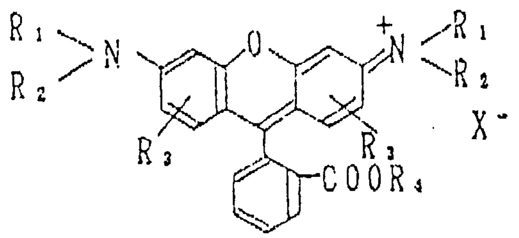

(V)

wherein $R_1$, and $R_2$ and $R_3$ have the above-described meanings, $R_4$ is a lower alkyl group, and X represents an anionic residue.

In the above case, when the esterification reaction is carried out using an alkylating agent, it is preferred from an industrial viewpoint that the organic solvent layer is subjected to the esterification reaction without having carried out the above-described extractive operation with acid.

As the alkylating agent, alkylsulfuric acid or alkyl halide is preferably used. Preferred examples thereof, include dimethylsulfuric acid, diethylsulfuric acid, dimethyl chloride, diethyl chloride, etc.

The work up of the ester compounds represented by the general formula (V) can be performed with an industrial advantage, for example, by the following method. As described above, the organic solvent layer is subjected to an esterification reaction using an alkylating agent and then, the esterification product thus obtained is subjected to steam distillation for water substitution of the solvent, and after filtration, washing, and drying the desired rhodamines are obtained. However, other recovery methods for these products may be used.

In accordance with the process of the present invention, even when the amount of the phthalic anhdride has been reduced to about 130-190% of the theoretical amount, almost no appreciable lowering in the yield and almost no increase in the by-product formation can be achieved by carrying out the reaction between the raw materials, phthalic anhydride and m-aminophenols represented by the above-described general formula (III) using the above-described specific organic solvents.

By carrying out the condensation reaction in an organic solvent of the present invention the amount of the phthalic anhydride is found to be sufficient in an amount of about 130-190 mole% of the theoretical, and further, by dividing the amount of diethyl-m-aminophenol being added into several portions, it has been found that even when the amount of the phthalic anhydride has been reduced to 130 mole% or less of the theoretical, the formation of N,N,N'-triethyl derivative can be suppressed to 1/2 - 1/3 of that of the prior art.

In the prior art, for instance, in the case of the synthesis of rhodamine B, when using the diethyl-m-aminophenol represented by the following formula (A):

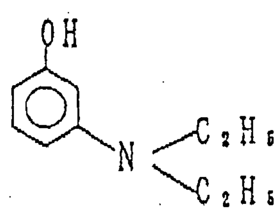

(A)

deethylation is liable to occur, so that at the time of the condensation reaction a N,N,N'-triethyl derivative represented by the following formula (B) or (C):

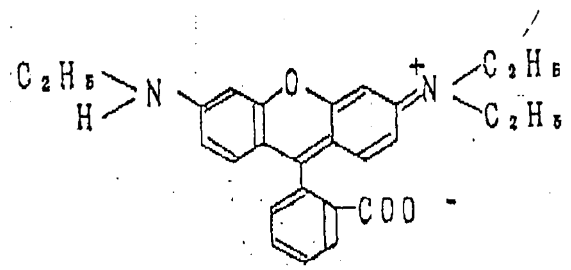

(B)

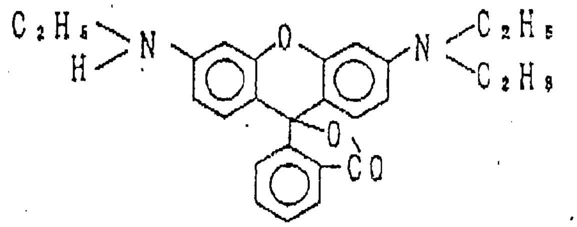

(C)

may be formed as the by-product, which not only lowers the yield but also remains in the product even after the subsequent procedures.

Further, in accordance with the process of the present invention, it was found that the excess phthalic anhydride remaining in the solvent can be readily extracted for removal into the aqueous systme by the extractive treatment with an aqueous solution of an alkali compound such as sodium hydroxide, etc., and then the dye material present in the solvent can be readily extracted into the aqueous system by the extractive treatment with, for instance, aqueous sulfuric acid or aqueous hydrochloric acid. Moreover, it was also found that in the above case the solvent layer from which the dye material has been extracted can be used as the solvent in the next condensation reaction and thus it was found that the process of the present invention is capable of industrialization.

In addition, the aqueous acidic solution as above is the same as the solution prior to crystallization in the prior art. Accordingly, the subsequent procedures can be same as in the prior art. That is to say, the compound represented by the above-described general formula (IV) can be separated by crystallization with acids and inorganic salts and also the compounds represented by the above-described general formula (II) can be obtained when crystallized by alkalizing with sodium hydroxide, etc. In this connection, the compounds represented by the above-described general formula (IV) can also be granulated according to the process described in Japanese Patent Kolai (OPI) No. 23,225/74.

In the conventional process, in order to obtain the esterification product represented by the above-described general formula (V) it has been absolutely necessary to once take out the compounds represented by the above-described general formulae (I) - (III) in the form of cake, whereas in the process of the present invention there is not always necessary, and it is possible to esterify either the condensation mixture as such or the condensation reaction mixture after the extractive treatment with an aqueous alkaline solution. Also, this esterification reaction mixture, after the reduced pressure distillation or steam distillation, and the like, can be crystallized out with an inorganic salt, etc. from the aqueous solution in the usual manner and separated as the dye cake.

In accordance with the process of the present invention the derived rhodamines can be produced with a high purity and in a high yield by the use of specific organic solvent(s) in the condensation reaction, and yet the amount of the raw material phthalic anhydride used can be remarkably reduced, and so on, so that the present invention can produce various excellent advantageous effects. Further, the organic solvents used can be recovered and reused.

Even in the case where an excess of phthalic anhydride is used, it can be readily removed by the solution by the solution extraction with an aqueous alkaline solution.

The procedure of obtaining a cake after the condensation reaction and the extractive treatment followed by washing in the prior art can be omitted by virtue of the procedures of the extraction of the dye material from the solvent with aqueous hydrochloric acide or aqueous sulfuric acid, etc. or water substitution by steam distillation, etc. Further, the compound to be esterified may be esterified a solvent system prior to the steam distillation.

The present invention will now be explained in more detail with reference to the following non-limiting Examples:

Example 1

Into a flask equiped with an agitating device, a separator for removing water from the system, and a condensor are added in order 170g of o-dichlorobenzene, 47g of phthalic anhydride, and 45g of diethyl-m-aminophenol, which are heated to 175°C while allowing nitrogen gas to flow in. After heating diethyl-m-amnophenol is added five times in 6g portions every one hour

After an additional three hours of reaction at the same temperature (during which reaction water is removed by means of the separator) the reaction liquid is discharged in 330g of 3% aqueous solution of sodium hydroxide, and after 30 minutes of agitation is allowed to stand for the liquids to separate. The organic solvent layer is mixed with 660g of 4.5% sulfuric acid for 30 minutes under agitation, and allowed to stand for the liquids to separate. The aqueous layer is agitated at 60°C, with the addition of 80g of 35% hydrochloric acid and 45g of sodium chloride, whereby rhodamine B represented by the following general formula:

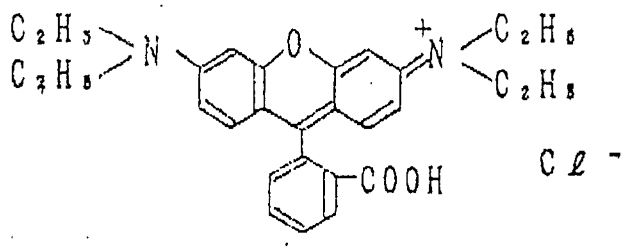

is obtained in the form of granules. It was filtered, washed with 300g of 2% hydrochloric acid, and dried.

Dry weight 102g, yield 93.7% (triethyl derivative (c) content about 1%)

Example 2

To the organic solvent (o-dichlorobenzene) which was recovered by liquid separation from Example 1, after mixing with 4.5% sulfuric acid was added frest O-dichlorobenzene up to a total amount of 170g, and by carrying out the same subsequent procedures as in Example 1, the same rhodamine B as in Example 1 was obtained.

Dry weight 103.6g, yield 95.2%

Example 3

In a flask equipped with a thermometer, an agitating device, a separator for removing water from the system and a condenser, are charged in order 170g of o-dichlorobenzene, 39.9g of phthalic anhydride, and 81.5g of o-ethylamino-p-cresol, which are heated to 180°C while sealing with nitrogen gas. After an additional three hours of reaction, the reaction mixture is cooled to 50°C, and after addition of 7.2g of sodium hydroxide, 70.5g of diethylsulfuric acid is added thereto dropwise over a period of one hour at a temperature of 40 - 55°C, and then the mixture is maintained at a temperature of 90 - 100°C for 5 hours.

Next, the seperator for removing the solvent from the system is affixed to the system, and, after addition of 1600g of hot water at 90°C, azeotropic distillation is carried out until no distillate of o-dichlorobenene is obtained. To the resulting liquid is added 35% hydrochloric acid to give a pH between 4 - 4.5, and after elevating the temperature to 80°C, 13.3g of 35% hydrochloric acid and 100g of sodium chloride are added thereto for crystallization. By filtering and drying the crystals thus obtained the rhodamine represented by the following general formula:

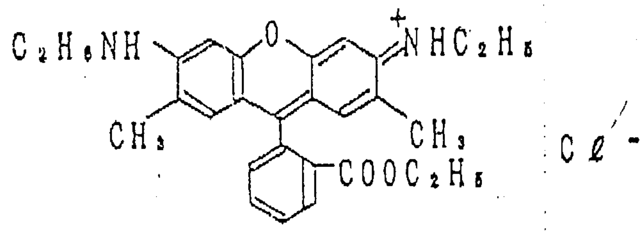

was obtained.

Dry weight 122.7g, yield 95.0%

Example 4

The solvent layer which was obtained by liquid separation from the condensation mixture in Example 1 is mixed with an aqueous solution of sodium hydroxide is cooled to 80°C, and after addition of 10g of sodium carbonate, 20g of dimethyl sulfuric acid is added thereto dropwise over a period of one hour, and then the whole is maintained at 90°C for three hours. Next, the separator for removing the solvent from the system is affixed to the system, and after addition of 310g of hot water at 90°C, azeotropic distillation is carried out until no distillate of o-dichlorobenzene is obtained. After 14g of 35% hydrochloric acid has been added thereto so as to once dissolve the residue, 7.5g of zinc chloride and 25.6g of sodium chloride are added for crystallization, and by filtering and drying the crystals thus obtained the rhodamine represented by the following general formula:

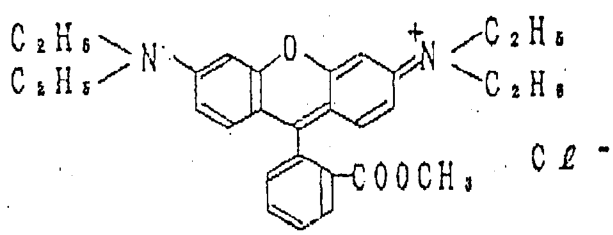

was obtained.

Dry weight 103.6g, yield 92.6%

Example 5

By carrying out the same procedures as in Exmaple 4, except that use was made of mineral spirit (industrial Gasoline No. 4) in place of o-dichlorbenzene in Example 4, the same rhodamine as in Example 4 was obtained.

Dru weight 105.4g, yield 94.2%

Comparative Example 1

In a four neck flask is charged 116g of phthalic anhydride, and is heated to 170°C. to melt it. While sealing with nitrogen gas, 90g of diethyl-m-aminophenom and 1.3g of 50° Be sulfuric acid are thrown thereinto at 180 - 185°C. over a period of 6 hours, and maintained at that temperature for three hours. Before the solidification begins, the reaction mixture is discharges in 384g of 11% aqueous solution of sodium hydroxide and, agitated at ambient temperature for 2 days.

The resulting dispersion is filtered and washed with 500g of water. The wet cake thus obtained is dissolved in 1200g of water and 30.4g of 35% hydrochloric acid, and filtered to remove the insoluble matter. The filtrate is heated to 60°C, and 52g of sodium chloride and 52g of 35% hydrochloric acid are added thereto for crystallization. The crystals thus obtained are filtered, washed with 2% hydrochloric acid, and dried.

There was obtained the same rhodamine B as in Example 1 in the form of granules.

Dry weight 116.2g, yield 89.0% (triethyl derivative (c) content about 3%)

Example 6

In a flask equiped with a thermometer, an agitating device, a separator for removing water from the system, and a condenser are charged in order 170g of o-chlorobenzene, 47g of phthalic anhydride, and 45g of diethyl-m-aminophenol and while allowing nitrogen gas to flow in, the temperature is elevated to 170°C, and while elevating the temperature up to 190°C gradually, diethyl-m-aminophenol is added five times in 6g portions every one hour.

After an additional three hours of reaction (during which reaction the water formed is removed by means of the separator), the reaction liquid is discharged in 330g of 3% aqueous solution of sodium hydroxide, and after 30 minutes of agitation, the whole is allowed to stand for liquid separation. The organic solvent layer is mixed with 660g of 4.5% sulfuric acid, and after 30 minutes of agitation, allowed to stand for liquid separation. The aqueous layer is agitated at 60°C, with the addition of 80g of 35% hydrochloric acid and 45g of sodium chloride, whereby rhodamine B represented by the following general formula:

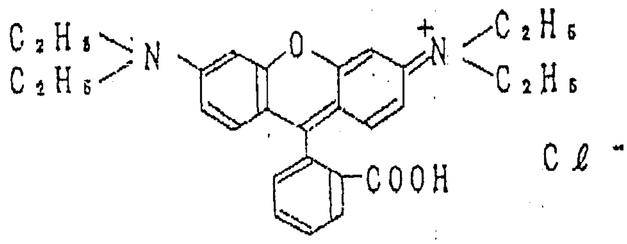

can be obtained in the form of granules. It was filtered, washed with 300g of 2% hydrochloric acid, and dried.

Dry weight 101g, yield 94.5% (triethyl derivative (C) content about 1%)

**Claims**

1. A process for preparation of a rhodamine represented by the following general formula (I) or (II):

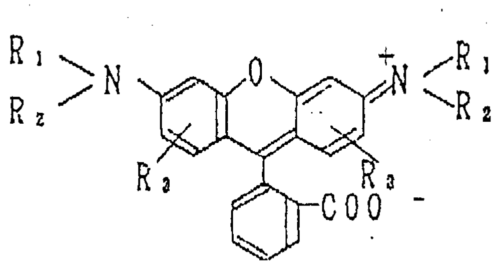

(I)

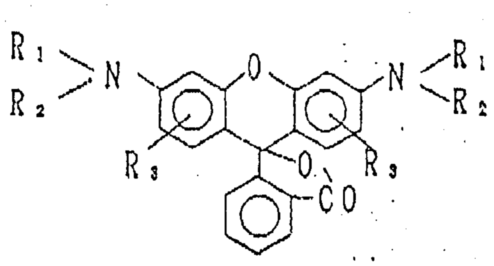

(II)

(wherein $R_1$, $R_2$, and $R_3$ are each independently hydrogen or a lower alkyl group, with the proviso that $R_1$ is not hydrogen when $R_2$ is hydrogen) and salts and esters thereof which comprises carrying out a condensation reaction with phthalic anhydride and N-substituted-m-aminophenols represented by the following general formula (III):

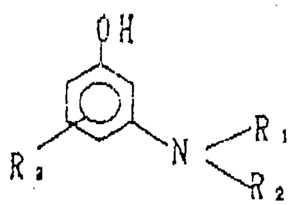

(III)

wherein $R_1$, $R_2$, and $R_3$ have the above-described meanings, in one or more organic solvents selected from halogenated and/or alkylated aromatic hydrocarbons or aliphatic hydrocarbons.

2. The process for preparing a rhodamine according to Claim 1, wherein the boiling point of said organic solvents is about 170°C or higher.

3. The process for preparing a rhodamine according to Claim 1 or 2, wherein the condensation reaction is carried out while removing the water out of the reaction system.

4. The process for preparing a rhodamine according to any preceding claim wherein the reaction mixture obtained by said condensation reaction is extracted with an aqueous alkali solution.

5. The process for preparing a rhodamine according to Claim 4, wherein said aqueous alkali solution is an aqueous solution of an inorganic alkali compound.

6. A process for preparing a rhodamine according to any preceding claim represented by the following general formula (IV):

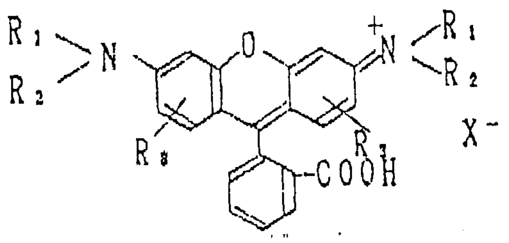

(IV)

(wherein $R_1$, $R_2$, and $R_3$ are as defined in Claim 1 and X represents an anionic residue,) which comprises treating a rhodamine obtained in accordance with the process of any one of Claims 1 to 5 with an aqueous acid solution.

7. The process for preparing a rhodamine according to Claim 6, wherein said aqueous acid solution is an aqueous solution of a mineral acid.

8. The process for preparing a rhodamine according to Claim 7, wherein said mineral acid is hydrochloric acid or sulfuric acid.

9. A process for preparing a rhodamine according to any preceding claim represented by the following general formula (V):

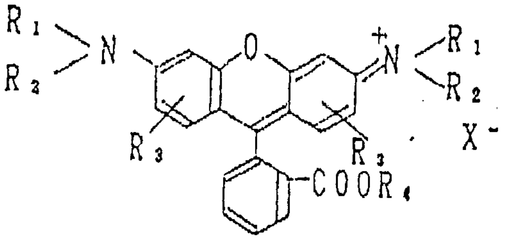

(V)

(wherein $R_1$, $R_2$, and $R_3$ are defined in Claim 1, $R_4$ represents a lower alkyl group, and X represents an

anionic residue), which comprises esterifying a rhodamine obtained from said condensation reaction with an alkylating agent.

**10.** The process for preparing a rhodamine as set forth in Claim 9, wherein said alkylating agent is alkylsulfuric acid or alkyl halide.

European Patent Office

# EUROPEAN SEARCH REPORT

Application Number
EP 91306886.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP - A1 - 0 184 114 (HODOGAYA CHEMICAL CO., LTD) * Claims 1,2 * -- | 1,2 | C 07 D 311/82<br>C 07 D 493/10 |
| A | EP - A1 - 0 333 649 (CIBA-GEIGY AG) * Claims 1,20 * -- | 1,2 | |
| A | US - A - 3 708 499 (ANDREE F. et al.) * Claims * -- | 1,2 | |
| A | CHEMICAL ABSTRACTS, vol. 112, no. 10, March 5, 1990, Columbus, Ohio, USA TOKUYAMA SODA CO., LTD. "Dental adhesive compositions containing vinyl monomers, fluores cains, and radical polymerization initiators" page 453, column 2, abstract-no. 84 272g & Jpn. Kokai Tokkyo Koho JP 01 158 086 (89 158 086) ---- | 1,2 | TECHNICAL FIELDS SEARCHED (Int. Cl.5)<br>C 07 D 311/00<br>C 07 D 493/00 |

The present search report has been drawn up for all claims

| Place of search | Date of completion of the search | Examiner |
|---|---|---|
| VIENNA | 19-09-1991 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)